# EUROPEAN PATENT SPECIFICATION

(11) **EP 1 545 360 B1**
(45) Date of publication and mention of the grant of the patent: **07.03.2007**
(21) Application number: 03752267.9
(22) Date of filing: 11.09.2003
(51) Int. Cl.: A61B 18/14

(54) **ELECTRODE ASSEMBLY FOR SEALING AND CUTTING TISSUE**
ELEKTRODENANORDNUNG ZUM VERSIEGELN UND SCHNEIDEN VON GEWEBE
ASSEMBLAGE D'ELECTRODE POUR SOUDER ET COUPER DES TISSUS

(30) Priority: 04.10.2002 US 416064 P
(43) Date of publication of application: 29.06.2005
(73) Proprietor: Sherwood Services AG, 8201 Schaffhausen (CH)
(72) Inventor: JOHNSON, Kristin, D., Louisville, CO 80027 (US); COUTURE, Gary, M., Longmont, CO 80501 (US)
(74) Representative: HOFFMANN EITLE
(86) International application number: PCT/US2003/028539
(87) International publication number: WO 2004/032777

(56) References cited:
- US-A- 5 833 690
- US-A1- 2002 107 517
- US-B1- 6 174 309

## Description

### BACKGROUND

The present disclosure relates to an electrode assembly for use with electrosurgical instruments and, more particularly, the present disclosure relates to an electrode assembly for use with an open or endoscopic electrosurgical forceps for sealing and cutting tissue. The technical features of the pre-characterizing part of claim 1 below are disclosed in combination in US-B-6174309.

### Technical Field

Open or endoscopic electrosurgical forceps utilize both mechanical clamping action and electrical energy to effect hemostasis. The electrode of each opposing jaw members is charged to a different electric potential such that when the jaw members grasp tissue, electrical energy can be selectively transferred through the tissue. A surgeon can either cauterize, coagulate/desiccate and/or simply reduce or slow bleeding, by controlling the intensity, frequency and duration of the electrosurgical energy applied between the electrodes and through the tissue.

To effectively seal tissue or vessels, especially thick tissue and large vessels, two predominant mechanical parameters must be accurately controlled: 1) the pressure applied to the vessel; and 2) the gap distance between the conductive tissue contacting surfaces (electrodes). As can be appreciated, both of these parameters are affected by the thickness of the vessel being sealed. Accurate application of pressure is important for several reasons: to oppose the walls of the vessel; to reduce the tissue impedance to a low enough value that allows enough electrosurgical energy through the tissue; to overcome the forces of expansion during tissue heating; and to contribute to the end tissue thickness which is an indication of a good seal. It has been determined that a typical fused vessel wall is optimum between 0.001 and 0.006 inches. Below this range, the seal may shred or tear and above this range the lumens may not be properly or effectively sealed.

With respect to smaller vessels, the pressure applied becomes less relevant and the gap distance between the electrically conductive surfaces becomes more significant for effective sealing. In other words, the chances of the two electrically conductive surfaces touching during activation increases as the tissue thickness and the vessels become smaller. However, the process of "coagulating" small vessels is fundamentally different than electrosurgically "sealing" vessels. For the purposes herein, "coagulation" is defined as a process of desiccating tissue wherein the tissue cells are ruptured and dried. "Vessel sealing" is defined as the process of liquefying the collagen in the tissue so that it reforms into a fused mass with significantly-reduced demarcation between the opposing tissue structures (opposing walls of the lumen). Coagulation of small vessels is usually sufficient to permanently close them. Larger vessels need to be sealed to assure permanent closure.

U.S. Patent No. 2,176,479 to Willis, U.S. Patent Nos. 4,005,714 and 4,031,898 to Hiltebrandt, U.S. Patent Nos. 5,827,274, 5,290,287 and 5,312,433 to Boebel et al., U.S. Patent Nos. 4,370,980, 4,552,143, 5,026,370 and 5,116,332 to Lottick, U.S. Patent No. 5,443,463 to Stern et al., U.S. Patent No. 5,484,436 to Eggers et al. and U.S. Patent No. 5,951,549 to Richardson et al., all relate to electrosurgical instruments for coagulating and cutting vessels or tissue. So far as is known, none of these designs provide uniformly reproducible pressure to the vessel or control the gap distance between electrically conductive surfaces and, thus, do not result in a uniform, consistent or effective seal.

Many of these instruments include blade members or shearing members which simply cut tissue in a mechanical and/or electromechanical manner and are relatively ineffective for vessel sealing purposes. Other instruments rely on clamping pressure alone to procure proper sealing thickness and are not designed to take into account gap tolerances and/or parallelism and flatness requirements which are parameters which, if properly controlled, can assure a consistent and effective tissue seal. For example, it is known that it is difficult to adequately control thickness of the resulting sealed tissue by controlling clamping pressure alone for either of two reasons: 1) if too much force is applied, there is a possibility that the two poles will touch and energy will not be transferred through the tissue resulting in an ineffective seal; or 2) if too low a force is applied the tissue may pre-maturely move prior to activation and sealing and/or a thicker, less reliable seal may be created.

Typically and particularly with respect to endoscopic electrosurgical procedures, once a vessel is sealed, the surgeon has to remove the sealing instrument from the operative site, substitute a new instrument through the cannula and accurately sever the vessel along the newly formed tissue seal. As can be appreciated, this additional step may be both time consuming (particularly when sealing a significant number of vessels) and may contribute to imprecise separation of the tissue along the sealing line due to the misalignment or misplacement of the severing instrument along the center of the tissue seal.

Several attempts have been made to design an instrument which incorporates a knife or blade member which effectively severs the tissue after forming a tissue seal. For example, U.S. Patent No. 5,674,220 to Fox et al. discloses a transparent instrument which includes a longitudinally reciprocating knife which severs the tissue once sealed. The instrument includes a plurality of openings which enable direct visualization of the tissue during the treatment and severing processes. This direct visualization allows a user to visually and manually regulate the closure force and gap distance between jaw members to reduce and/or limit certain undesirable visual effects known to occur when treating vessels, thermal spread, charring, etc. As can be appreciated, the overall success of creating an effective tissue seal with this instrument is greatly reliant upon the user's expertise, vision, dexterity, and experience in judging the appropriate closure force, gap distance and length of reciprocation of the knife to uniformly, consistently and effectively seal the vessel and separate the tissue at the seal along an ideal cutting plane.

U.S. Patent No. 5,702,390 to Austin et al. discloses an instrument which includes a triangularly-shaped electrode which is rotatable from a first position to treat tissue to a second position to cut tissue. Again, the user must rely on direct visualization and expertise to control the various effects of treating and cutting tissue.

Thus, a need exists to develop an electrosurgical instrument which includes an electrode assembly which enables the surgeon to both seal the tissue in an effective and consistent manner and subsequently separate the tissue along the tissue seal without re-grasping the tissue or removing the instrument from the operating cavity.

### SUMMARY

The present invention is defined in claim 1 below. The dependent claims are directed to optional or preferred features.

The present disclosure relates to an electrode assembly for use with an instrument for sealing vessels. The electrode assembly includes a pair of opposing first and second jaw members which are movable relative to one another from a first position wherein the jaw members are disposed in spaced relation relative to one another to a second position wherein the jaw members cooperate to grasp tissue therebetween. Each jaw member includes an electrically conductive tissue contacting surface which is connected to an electrosurgical generator (ESU) such that the tissue contacting surfaces are capable of conducting electrosurgical energy through the tissue held therebetween to effect a tissue seal.

Advantageously, the first jaw member includes an electrically conductive cutting element dielectrically disposed within the first tissue contacting surface and the second jaw member includes an insulator disposed therein opposite the cutting element. The cutting element extends outwardly from the first tissue contacting surface towards the second tissue contacting surface to create a gap between the tissue contacting surfaces during sealing. One advantage of the present invention is that the cutting element and the first tissue contacting surface are independently activatable by the surgeon to selectively "cut" or "seal" the tissue, respectively. Another advantage of the present invention is that the cutting element is substantially dull and only capable of cutting tissue through electrosurgical activation.

In one advantageous embodiment, the electrode assembly includes a smart sensor for determining overall seal quality prior to activating the cutting element. The smart sensor may employ an audible or visual indicator depending upon a particular purpose to provide feedback to the surgeon regarding overall seal quality. It may be advantageous to have the smart sensor determine seal quality by measuring the drop in current across the tissue, by measuring the tissue impedance across the tissue and/or by measuring the tissue temperature or moisture content of the tissue.

In another particularly advantageous embodiment, the same or a second smart sensor may be employed to measure or determine various tissue parameters to regulate / control the electrosurgical energy supplied to the cutting element during cutting. Preferably, after a tissue seal is created, the smart sensor measures at least one of tissue thickness, tissue moisture, tissue density and/or tissue impedance to regulate / control the electrosurgical energy supplied to the cutting element during cutting. Advantageously, the ESU can be designed to send a calibrating pulse through the tissue which enables the smart sensor to measures various tissue parameters to control the electrosurgical energy supplied to the cutting element during cutting.

Yet, in another advantageous embodiment according to the present disclosure, a smart sensor is employed to automatically switch electrosurgical energy to the cutting element once the tissue is sealed. In still yet another particularly useful embodiment, the ESU is configured to deliver electrosurgical energy in a pulse-like manner to effect at least one of cutting and sealing. Another advantageous embodiment employs a smart sensor which includes a variable resistor which automatically regulates the electrosurgical energy from the ESU during both the sealing and cutting processes.

The present description includes disclosure of a method for sealing and cutting tissue and includes the steps of: providing an electrode assembly having a pair of opposing first and second jaw members which are movable relative to one another from a first position wherein the jaw members are disposed in spaced relation relative to one another to a second position wherein the jaw members cooperate to grasp tissue therebetween. Preferably, each of the jaw members includes an electrically conductive tissue contacting surface which is connected to the ESU.

An electrically conductive cutting element is dielectrically disposed within the first tissue contacting surface. The cutting element extends from the first tissue contacting surface towards the second tissue contacting surface. Preferably, when the tissue contacting surfaces are closed about the tissue, the cutting element creates a gap "G" between the first and second tissue contacting surfaces. An insulator is disposed within the second tissue contacting surface opposite the cutting element.

The method further includes the steps of: actuating the jaw members to grasp tissue between tissue contacting surfaces; applying a closure force "F' between tissue contacting surfaces such that the cutting element creates a gap "G" between the first and second tissue contacting surfaces; energizing the first and second tissue contacting surfaces to deliver electrosurgical energy through the tissue to effect a tissue seal; and energizing the cutting element and the second tissue contacting surface to deliver electrosurgical energy through the tissue to effectively cut the tissue along the tissue seal.

Alternatively, after the step of energizing the first and second tissue contacting surfaces to effect a tissue seal, the method includes the step of: utilizing a smart sensor to determine seal quality prior to cutting the tissue.

### BRIEF DESCRIPTION OF THE DRAWINGS

Various embodiments of the subject instrument are described herein with reference to the drawings wherein:
Fig. 1A is a left, perspective view of an endoscopic bipolar forceps showing a housing, a shaft and an electrode assembly according to the present disclosure;
Fig. 1B is a left, perspective view of an open bipolar forceps having an electrode assembly according to the present disclosure;
Fig. 2 is an enlarged view of a selectively detachable electrode assembly according to the present disclosure;
Fig. 3A is a schematic, front view showing a first jaw member having a tissue contacting surface with a cutting element disposed therein and a second jaw member having a tissue contacting surface with an insulator disposed therein opposite the cutting element;
Fig. 3B is a schematic, front view showing the tissue contacting surfaces and their respective electrical connections to a switch controller, a smart sensor and/or an electrosurgical generator;
Fig. 3C is a schematic, front view showing alternate electrical connections to the switch controller and the electrosurgical generator;
Fig. 4A is a schematic, front view showing tissue being sealed between tissue contacting surfaces through a combination of clamping the tissue in a particular pressure range, maintaining a desired gap range between opposing tissue contacting surfaces during sealing and applying a predetermined amount of electrosurgical energy through the tissue;
Fig. 4B is a schematic, front view showing tissue being cut by the cutting element through a combination of applying a cutting pressure within a particular range and applying electrosurgical energy from the cutting element through the tissue;
Fig. 4C is an enlarged, schematic view of Fig. 4B showing the path of the electrosurgical current from the cutting element, through the tissue and to the second tissue contacting surface;
Fig. 5A is a schematic, front view of an alternate jaw configuration for the electrode assembly which lacks the extending cutting element of claim 1 below;
Fig. 5B is a finite element analysis model of showing the current density during cutting utilizing the alternate jaw member configuration of Fig. 5A; and
Figs. 6A and 6B are schematic views of an alternate embodiment of the present disclosure wherein the polarities of the electrodes are changed to effect tissue cutting.

### DETAILED DESCRIPTION

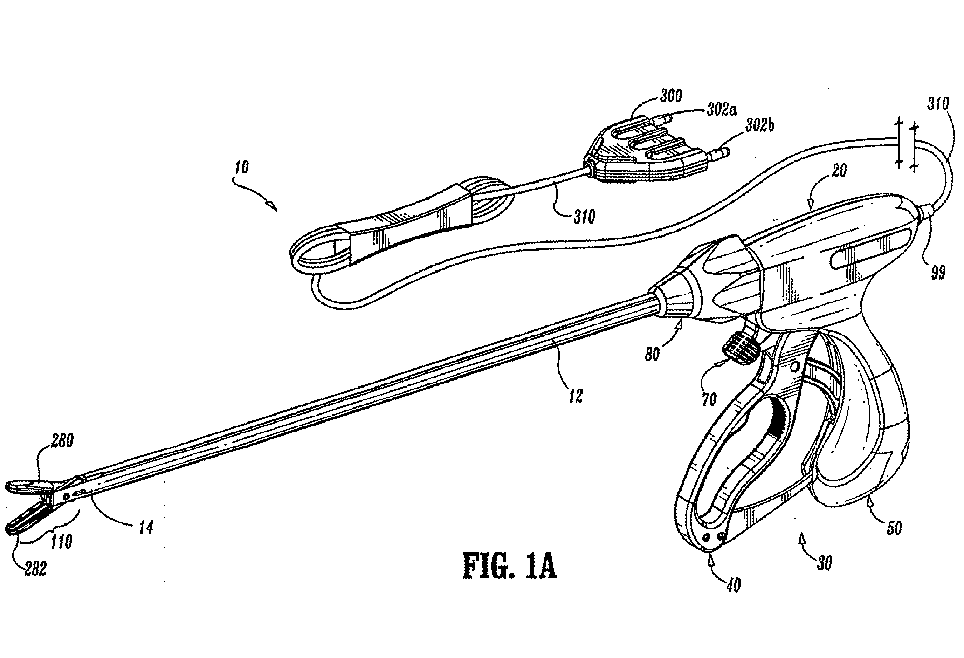

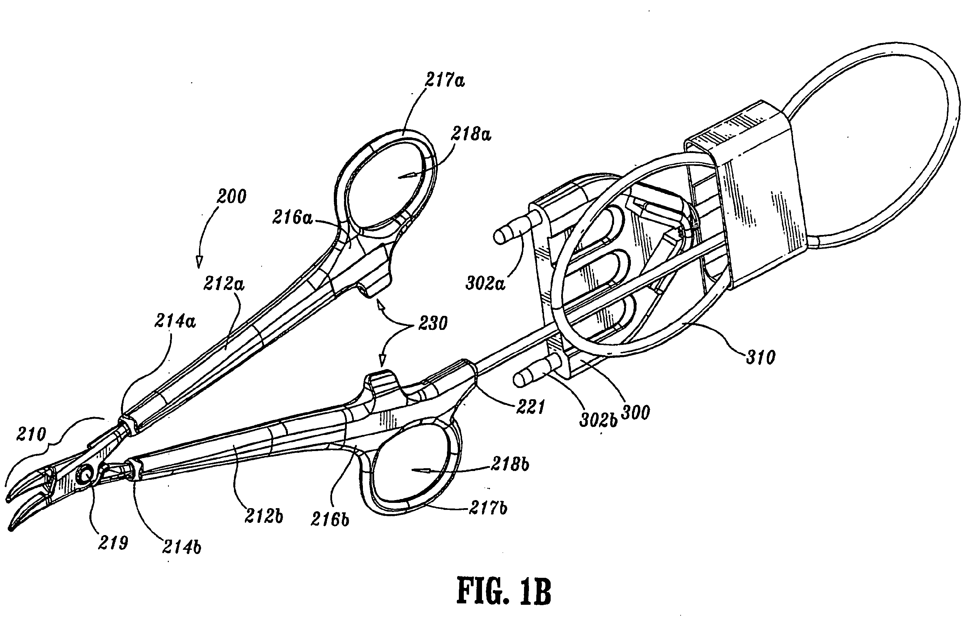

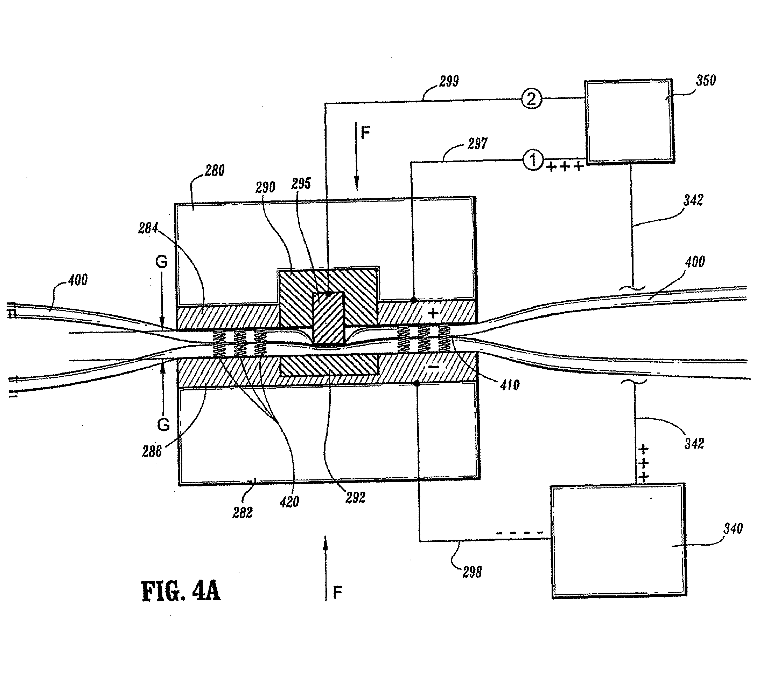

Referring now to Figs. 1 A and 1 B, a bipolar forceps 10 is shown for use with various surgical procedures. Forceps 10 generally includes a housing 20, a handle assembly 30, a rotating assembly 80, a trigger assembly 70 and an electrode assembly 110 which mutually cooperate to grasp, seal and divide tubular vessels and vascular tissue 400 (See Fig. 4A). Although the majority of the figure drawings depict a bipolar forceps 10 for use in connection with endoscopic surgical procedures, an open forceps 200 is also contemplated for use in connection with traditional open surgical procedures and is shown by way of example in Fig. 1 B and is described below. For the purposes herein, either an endoscopic instrument or an open instrument may be utilized with the electrode assembly described herein. Obviously, different electrical and mechanical connections and considerations apply to each particular type of instrument, however, the novel aspects with respect to the electrode assembly and its operating characteristics remain generally consistent with respect to both the open or endoscopic designs.

More particularly, forceps 10 includes a shaft 12 which has a distal end 14 dimensioned to mechanically engage the electrode assembly 110 and a proximal end 16 which mechanically engages the housing 20. The shaft 12 may be bifurcated at the distal end 14 thereof to receive the electrode assembly 110. The proximal end 16 of shaft 12 mechanically engages the rotating assembly 80 to facilitate rotation of the electrode assembly 110. In the drawings and in the descriptions which follow, the term "proximal", as is traditional, will refer to the end of the forceps 10 which is closer to the user, while the term "distal" will refer to the end which is further from the user.

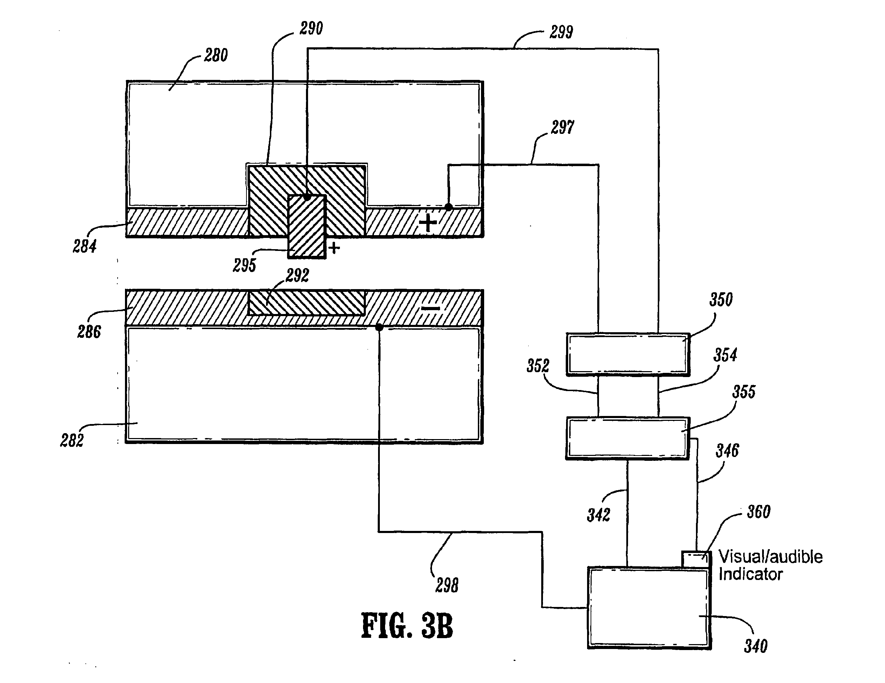

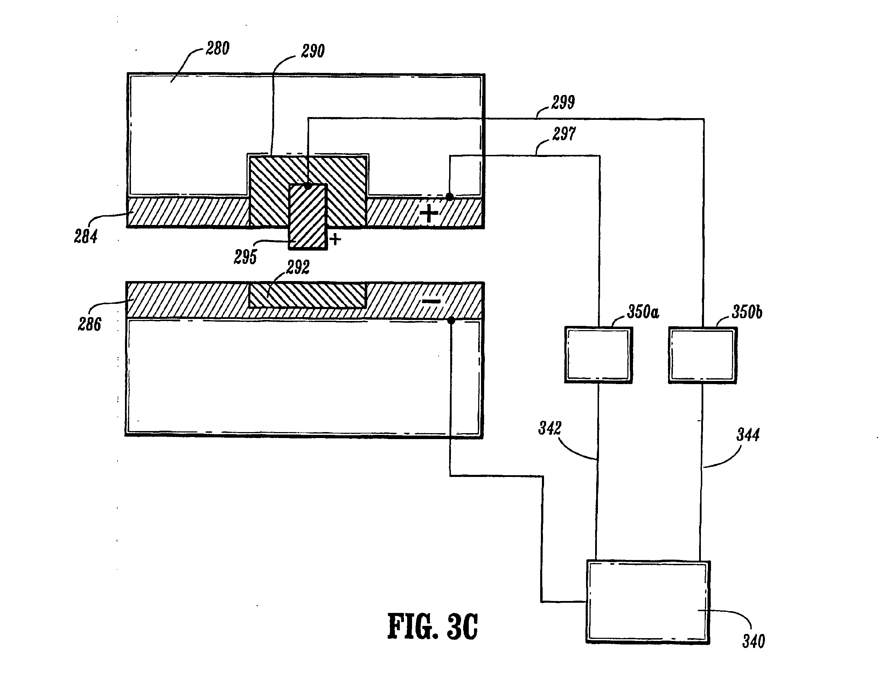

As best seen in Fig. 1 A, forceps 10 also includes an electrical interface or plug 300 which connects the forceps 10 to a source of electrosurgical energy, e.g., an electrosurgical generator 340 (See Fig. 3B). Plug 300 includes a pair of prong members 302a and 302b which are dimensioned to mechanically and electrically connect the forceps 10 to the electrosurgical generator 340. An electrical cable 310 extends from the plug 300 to a sleeve 99 which securely connects the cable 310 to the forceps 10. Cable 310 is internally divided within the housing 20 to transmit electrosurgical energy through various electrical feed paths to the electrode assembly 110.

Handle assembly 30 includes a fixed handle 50 and a movable handle 40. Fixed handle 50 is integrally associated with housing 20 and handle 40 is movable relative to fixed handle 50 to actuate a pair of opposing jaw members 280 and 282 of the electrode assembly 110 as explained in more detail below. Movable handle 40 and trigger assembly 70 are preferably of unitary construction and are operatively connected to the housing 20 and the fixed handle 50 during the assembly process.

As mentioned above, electrode assembly 110 is attached to the distal end 14 of shaft 12 and includes a pair of opposing jaw members 280 and 282. Movable handle 40 of handle assembly 30 imparts movement of the jaw members 280 and 282 from an open position wherein the jaw members 280 and 282 are disposed in spaced relation relative to one another, to a clamping or closed position wherein the jaw members 280 and 282 cooperate to grasp tissue 400 therebetween (See Fig. 4A).

It is envisioned that the forceps 10 may be designed such that it is fully or partially disposable depending upon a particular purpose or to achieve a particular result. For example, electrode assembly 110 may be selectively and releasably engageable with the distal end 14 of the shaft 12 and/or the proximal end 16 of shaft 12 may be selectively and releasably engageable with the housing 20 and the handle assembly 30. In either of these two instances, the forceps 10 would be considered "partially disposable" or "reposable", i.e., a new or different electrode assembly 110 (or electrode assembly 110 and shaft 12) selectively replaces the old electrode assembly 110 as needed.

Referring now to Figs. 1 B, an open forceps 200 includes a pair of elongated shaft portions 212a each having a proximal end 216a and 216b, respectively, and a distal end 214a and 214b, respectively. The forceps 200 includes electrode assembly 210 which attaches to distal ends 214a and 214b of shafts 212a and 212b, respectively. Electrode assembly 210 includes opposing jaw members 280 and 282 which are pivotably connected about a pivot pin 219.

Preferably, each shaft 212a and 212b includes a handle 217a and 217b disposed at the proximal end 216a and 216b thereof which each define a finger hole 218a and 218b, respectively, therethrough for receiving a finger of the user. As can be appreciated, finger holes 218a and 218b facilitate movement of the shafts 212a and 212b relative to one another which, in turn, pivot the jaw members 280 and 282 from an open position wherein the jaw members 280 and 282 are disposed in spaced relation relative to one another to a clamping or closed position wherein the jaw members 280 and 282 cooperate to grasp tissue 400 therebetween. A ratchet 230 is preferably included for selectively locking the jaw members 280 and 282 relative to one another at various positions during pivoting.

Preferably, each position associated with the cooperating ratchet interfaces 230 holds a specific, i.e., constant, strain energy in the shaft members 212a and 212b which, in turn, transmits a specific closing force to the jaw members 280 and 282. It is envisioned that the ratchet 230 may include graduations or other visual markings which enable the user to easily and quickly ascertain and control the amount of closure force desired between the jaw members 280 and 282.

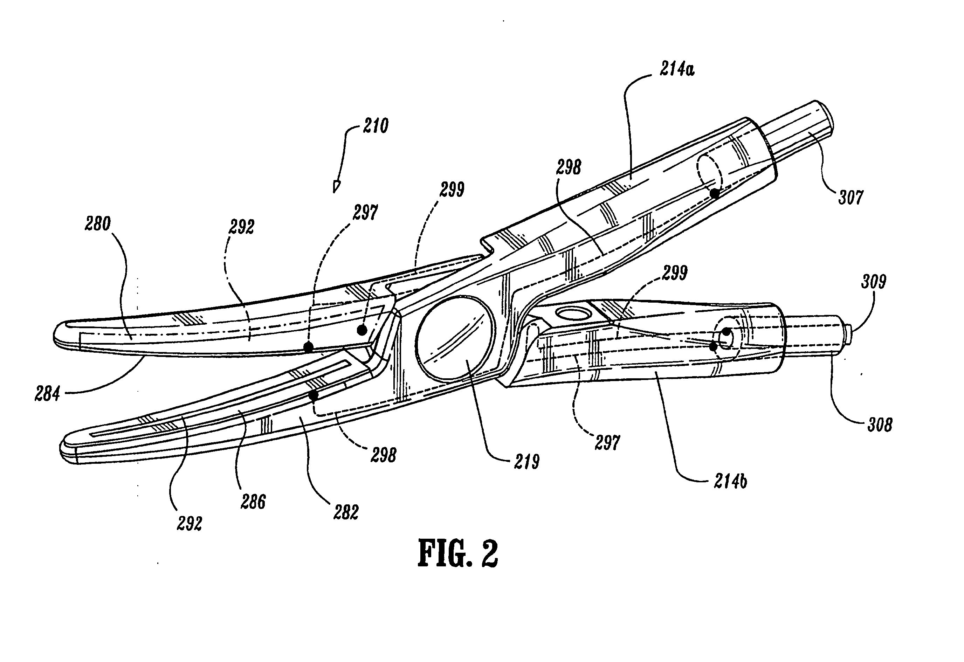

One of the shafts, e.g., 212b, includes a proximal shaft connector /flange 221 which is designed to connect the forceps 200 to a source of electrosurgical energy such as an electrosurgical generator 342. More particularly, flange 221 mechanically secures electrosurgical cable 310 to the forceps 200 such that the user may selectively apply electrosurgical energy as needed. The proximal end of the cable 310 includes a similar plug 300 as described above with respect to Fig. 1 A. The interior of cable 310 houses a pair of leads which conduct different electrical potentials from the electrosurgical generator 340 to the jaw members 280 and 282 as explained below with respect to Fig. 2.

Preferably, the jaw members 280 and 282 are generally symmetrical and include similar component features which cooperate to permit facile rotation about pivot 219 to effect the grasping and sealing of tissue 400. Each jaw member 280 and 282 includes an electrically conductive tissue contacting surface 284 and 286, respectively, which cooperate to engage the tissue 400 during sealing and cutting. One of the jaw members includes a cutting element 295 disposed therein which is explained in detail below.

As best shown in Fig. 2, the various electrical connections of the electrode assembly 210 are preferably configured to provide electrical continuity to the tissue contacting surfaces 284 and 286 and the cutting element 295 through the electrode assembly 210. More particularly, two connector pins 307 and 308 are located at the proximal end of the electrode assembly 210. Connections 307 and 308 are preferably mechanically and electrically interfaced with corresponding electrical connections (not shown) disposed within shafts 212a and 212b, respectively. As can be appreciated, the electrical connectors 307 and 308 may be permanently soldered to the shafts 212a and 212b during the assembly process of a disposable instrument or, alternatively, selectively removable for use with a reposable instrument.

Connector 307 is internally connected to lead 298 disposed within the electrode assembly 210 to provide electrical continuity to tissue contacting surface 286 of jaw member 282. Likewise, connector 308 is internally connected to lead 297 which provides electrical continuity to tissue contacting surface 284 of jaw member 280. Connector . 308 also includes a second electrical interface 309 which provides electrical continuity to the cutting element 295 through electrode assembly 210. Interfaces 308 and 309 are preferably dielectrically insulated from one another to allow selective and independent activation of either the tissue contacting surface 284 or the cutting element 295.

Preferably, the leads 297, 298 and 299 (and/or conductive pathways) do not encumber the movement of the jaw members 280 and 282 relative to one another during the manipulation and grasping of tissue 400. Likewise, the movement of the jaw members 280 and 282 do not unnecessarily strain the lead connections.

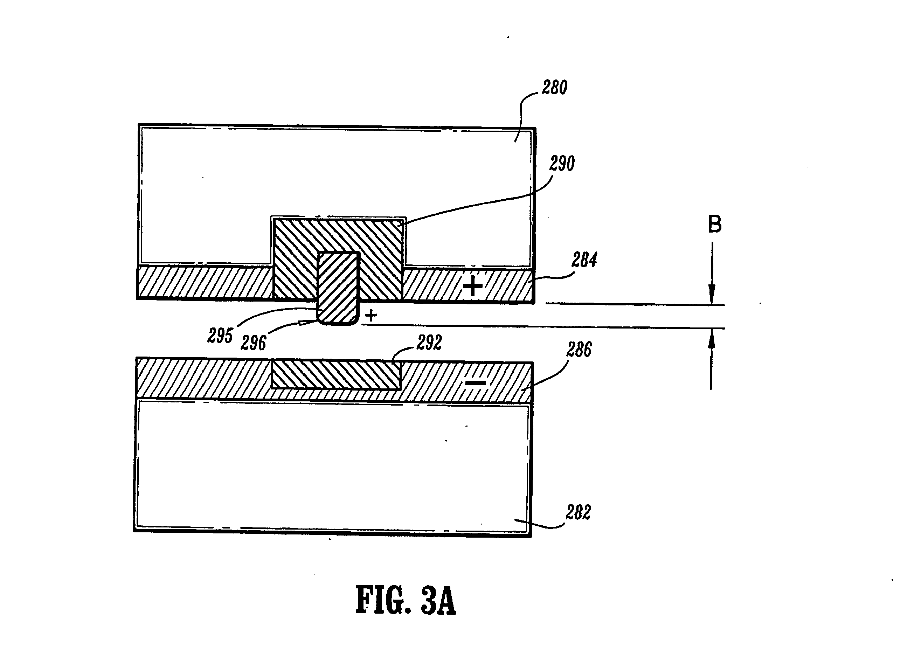

As best seen in Figs. 2 and 3A-3B jaw members 280 and 282 both include conductive tissue contacting surfaces 284 and 286, respectively, disposed along substantially the entire longitudinal length thereof (i.e., extending substantially from the proximal to distal end of the respective jaw member 280 and 284). It is envisioned that tissue contacting surfaces 284 and 286 may be attached to the jaw member 280, 284 by stamping, by overmolding, by casting, by overmolding a casting, by coating a casting, by overmolding a stamped electrically conductive sealing plate and/or by overmolding a metal injection molded seal plate. All of these manufacturing techniques can be employed to produce jaw member 280 and 284 having electrically conductive tissue contacting surfaces 284 and 286 disposed thereon for contacting and grasping tissue.

Preferably, the electrically conductive sealing surfaces 284 and 286 may also include a pinch trim (not shown) which facilitates secure engagement of the electrically conductive surfaces 284 and 286 to the jaw members 280 and 282 and also simplifies the overall manufacturing process. It is envisioned that each electrically conductive sealing surface 284, 286 may also include an outer peripheral edge which has a radius and the respective jaw member 280, 282 meets the electrically conductive sealing surface 284, 286 along an adjoining edge which is generally tangential to the radius and/or meets along the radius.

The electrically conductive tissue contacting surfaces 284 and 286 of the jaw members 280 and 282 both include an insulator or insulative material 290 and 292, respectively, disposed substantially along the entire longitudinal length thereof. Each insulator 290 and 292 is generally centered across the width of its respective tissue contacting surface 284 and 286, respectively, along substantially the entire length of the tissue contacting surface 284 and 286 such that the two insulators 290 and 292 generally oppose one another.

Preferably, one or both of the insulators 290, 292 is made from a ceramic material due to its hardness and inherent ability to withstand high temperature fluctuations. Alternatively, one or both of the insulators 290, 292 may be made from a material having a high Comparative Tracking Index (CTI) having a value in the range of about 300 to about 600 volts. Examples of high CTI materials include nylons and syndiotactic polystryrenes such as QUESTRA^{®} manufactured by DOW Chemical. Other materials may also be utilized either alone or in combination, e.g., Nylons, Syndiotactic-polystryrene (SPS), Polybutylene Terephthalate (PBT), Polycarbonate (PC), Acrylonitrile Butadiene Styrene (ABS), Polyphthalamide (PPA), Polymide, Polyethylene Terephthalate (PET), Polyamide-imide (PAl), Acrylic (PMMA), Polystyrene (PS and HIPS), Polyether Sulfone (PES), Aliphatic Polyketone, Acetal (POM) Copolymer, Polyurethane (PU and TPU), Nylon with Polyphenylene-oxide dispersion and Acrylonitrile Styrene Acrylate.

Jaw member 280 includes an electrically conductive cutting element 295 disposed substantially within the insulator 190. As described in detail below, the cutting element 295 plays a dual role during the sealing and cutting processes. Preferably, the entire cutting element 295 is electrically conductive, however, it is envisioned that the cutting element 295 may be made from an insulative material with a conductive coating disposed thereon. Cutting element 295 is configured to extend from insulator 290 and the tissue contacting surface 284 a distance "B" (See Fig. 3A) such that the cutting element 295 acts as a gap stop (i.e., creates a gap distance "G" (See Fig. 4A) between conductive sealing surfaces 284 and 286) which promotes accurate, consistent and effective tissue sealing. As can be appreciated, the cutting element 295 also prevents the two tissue contacting surfaces 284 and 286 from touching which eliminates the chances of the instrument shorting during sealing.

As mentioned above, two mechanical factors play an important role in determining the resulting thickness of the sealed tissue and effectiveness of a tissue seal 410, i.e., the pressure applied between opposing jaw members 280 and 282 and the gap distance "G" between the opposing tissue contacting surfaces 284 and 286 during the sealing process. Preferably, the cutting element 295 extends from the tissue contacting surface 284 a predetermined distance "B" according to the specific material properties (e.g., _ compressive strength, thermal expansion, etc.) to yield a consistent and accurate gap distance "G" during sealing (Fig. 4A). Preferably, the gap distance "G" during sealing ranges from about 25 to about 150µm (about 0.001 inches to about 0.006 inches) and, more preferably, between about 50 and about 75µm (about 0.002 inches and about 0.003 inches). The clamping pressure between the opposing tissue contacting surfaces 284 and 286 is preferably between about 3 kg/cM² to about 16 kg/cm². Obviously, the pressure between the cutting element 295 and the opposite insulator 292 is much higher due to the smaller surface area of the cutting element 295 against the insulator 292.

As best seen in Fig. 3A, the conductive cutting element 295 is oriented in opposing, vertical registration with the insulator 292 of jaw member 282. It is envisioned that the cutting element 295 is substantially dull which, as can be appreciated, does not inhibit the sealing process (i.e., premature cutting) during initial electrosurgical activation. In other words, the surgeon is free to manipulate, grasp and clamp the tissue 400 for sealing purposes without the cutting element 295 mechanically cutting into the tissue 400. Moreover, tissue cutting can only be achieved through a combination of mechanically clamping the tissue between the cutting element 295 and the opposing insulator 292 and applying electrosurgical energy from the cutting element 295, through the tissue 400 and to the return electrode, i.e., the electrically conductive tissue contacting surface 286.

It is envisioned that the geometrical configuration of the cutting element 295 plays an important role in determining the overall effectiveness of tissue cut. For example, the power/current concentration around the cutting element 295 is based upon the particular geometrical configuration of the cutting element 295 and the cutting element's 295 proximity to the return electrode, i.e., tissue contacting surface 286. Certain geometries on the cutting element create areas of high power/current concentration. Moreover, the spacing of the return electrode 286 to these power/current concentrations effects the electrical fields through the tissue 400. Therefore, by configuring the cutting element 295 and insulator 292 within close proximity to one another the electrical current field remains high which is ideal for cutting but the instrument will not short due to accidental contact between conductive surfaces. As can be appreciated, the relative size of the cutting element 295 and/or the size of the insulator 292 may be selectively altered to accomplish this purpose.

Figs. 3B and 3C shows schematic examples of how the tissue contacting surfaces 284 and 286 and the cutting element 295 may be electrically coupled to an electrosurgical generator 340 (ESU) and switch controllers. More particularly, Fig. 3B shows an electrical lead 297 extending from tissue contacting surface 284 and an electrical lead 298 extending from tissue contacting surface 286. More particularly, lead 297 couples the tissue contacting surface 284 to a switch 350, lead 352 couples the switch 350 to a smart sensor 355 and lead 342 couples the smart sensor 355 to the ESU 340, respectively. Lead 298 couples the tissue contacting surface 286 directly to the ESU 340. With respect to the cutting element 295, lead 299 couples the cutting element 295 to switch 350, lead 354 couples the switch 350 to the smart sensor 355 and lead 342 couples the smart sensor 355 to the ESU 340, respectively.

As can be appreciated, this electrical arrangement allows the surgeon to initially activate the two opposing tissue contacting surfaces 284 and 286 to seal the tissue 400 and, subsequently, selectively and independently activate the cutting element 295 and tissue contacting surface 286 to cut the tissue 400. Hence, the tissue 400 is initially sealed and thereafter cut without re-grasping the tissue.

However, it is envisioned that the cutting element 295 and the tissue contacting surface 286 may also be activated to simply cut tissue/vessels without initially sealing. For example, the jaw members 280 and 282 may be opened and the cutting element 295 may be selectively activated to dissect or coagulate tissue 400. This type of alternative embodiment may be particularly useful during certain endoscopic procedures wherein an electrosurgical pencil is typically introduced to coagulate and/or dissect tissue during the operating procedure.

A switch 350 may be employed to allow the surgeon to selectively activate tissue contacting surface 284 or the cutting element 295 independently of one another. As can be appreciated, this allows the surgeon to initially seal tissue 400 and then activate the cutting element 295 by simply turning the switch 350. Rocker switches, toggle switches, flip switches, dials, etc. are types of switches which can be commonly employed to accomplish this purpose. It is also envisioned that the switch 350 may cooperate with the smart sensor 355 (or smart circuit, computer, feedback loop, etc.) which automatically triggers the switch 350 to change between the "sealing" mode and the "cutting" mode upon the satisfaction of a particular parameter. For example, the smart sensor 355 may include a feedback loop which indicates when a tissue seal is complete based upon one or more of the following parameters: tissue temperature, tissue impedance at the seal 410, change in impedance of the tissue over time and/or changes in the power or current applied to the tissue over time. An audible or visual feedback monitor 360 may be employed to convey information to the surgeon regarding the overall seal quality or the completion of an effective tissue seal 410. A separate lead 346 may be connected between the smart sensor 355 and the ESU for visual and/or audible feedback purposes.

Preferably, the ESU 340 delivers energy to the tissue in a pulse-like waveform. It has been determined that delivering the energy in pulses increases the amount of sealing energy which can be effectively delivered to the tissue and reduces unwanted tissue effects such as charring. Moreover, the feedback loop of the smart sensor 355 can be configured to automatically measure various tissue parameters during sealing (i.e., tissue temperature, tissue impedance, current through the tissue) and automatically adjust the energy intensity and number of pulses as needed to reduce various tissue effects such as charring and thermal spread.

It has also been determined that RF pulsing may be used to more effectively cut tissue. For example, an initial pulse from the cutting element 295 through the tissue 400 (or the tissue contacting surfaces 284 and 286 through the tissue 400) may be delivered to provide feedback to the smart sensor 355 for selection of the ideal number of subsequent pulses and subsequent pulse intensity to effectively and consistently cut the amount or type of tissue 400 with minimal effect on the tissue seal 410. It is believed that electrical cutting occurs in spikes which are generally within the first 0.01 seconds to 0.5 seconds of energy application. If the energy is not pulsed, the tissue may not initially cut but desiccate since tissue impedance remains high during the initial stages of cutting. By providing the energy in short, high energy pulses, it has been found that the tissue 400 is more likely to cut.

Alternatively, switch 350 may be configured to activate based upon a desired cutting parameter and/or after an effective seal is created or has been verified. For example, after effectively sealing the tissue 400, the cutting element 295 may be automatically activated based upon a desired end tissue thickness at the seal 410.

Fig. 3C shows another alternative embodiment of the present disclosure which utilizes two active switches 350a and 350b to separately and independently energize the conductive elements, i.e., tissue contacting surface 284 and cutting element 295. As such, the surgeon actively depresses switch 350a to effect sealing and switch 350b to effect cutting. Tissue contacting surface 286 is directly coupled to the ESU 340 and acts as the return electrode in either instance.

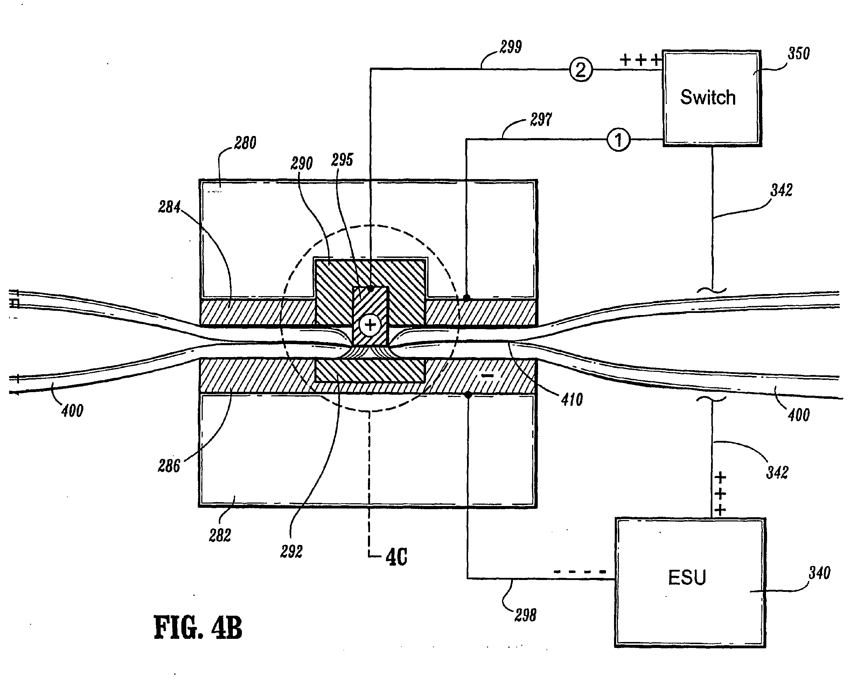

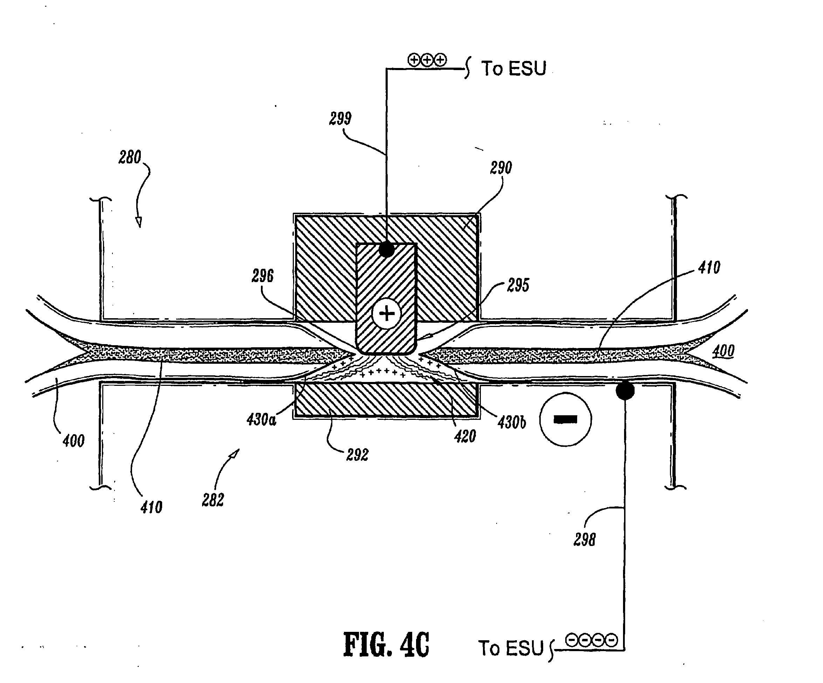

Turning now to the operation of the electrosurgical instrument 10 (or 200), Fig. 4A shows the pathway of the electrosurgical energy 420 from the opposing conductive surfaces 284 and 286 and through the tissue 400 during the sealing process and Figs. 4B and 4C show the pathway of the electrosurgical energy 420 from the cutting element 295 and through the tissue 400 during the cutting process. More particularly, Fig. 4A shows the tissue 40 being compressed between the conductive tissue contacting surfaces 284 and 286 of the jaw member 280 and 282 under a closure pressure "F" in the range of about 3 kg/cm² to about 16 kg/cm². Applying the correct force "F" is also important for other reasons: to oppose the walls of the vessel; to reduce the tissue impedance to a low enough value that allows enough current through the tissue 400; and to overcome the forces of expansion during tissue heating in addition to contributing towards creating the required end tissue thickness which is an indication of a good tissue seal 410. Tissue pressures within a working range of about 7 kg/cm² to about 13 kg/cm² have been shown to be particularly effective for sealing arteries and vascular bundles.

Upon compression, the cutting element 295 acts as a stop member and creates a gap "G" between the opposing conductive tissue contacting surfaces 284 and 286. Preferably, the gap distance is in the range of about 25 to about 150 µm (about 0.001 to about 0.0006 inches). As mentioned above, controlling both the gap distance "G" and clamping pressure "F" between conductive surfaces 284 and 286 are two important mechanical parameters which need to be properly controlled to assure a consistent and effective tissue seal. The surgeon activates the ESU 340 and the switch 350 (either manually or automatically as described above) to transmit electrosurgical energy 420 to the tissue contacting surfaces 284 and 286 and through the tissue 400 to effect a seal 410. As a result of the unique combination of the clamping pressure "F", gap distance "G" and electrosurgical energy 420, the tissue collagen melts into a fused mass with limited demarcation between opposing vessel walls.

Once sealed, the surgeon activates the cutting element 295 as illustrated in Figs. 4B and 4C. More particularly, the surgeon activates the switch 350 to energize the cutting element 295 to cut the tissue 400. As mentioned above, the surgeon does not necessarily need to re-grasp the tissue 400 to cut, i.e., the cutting element 295 is already positioned proximate the ideal, center cutting line of the seal. As best seen in Fig. 4C, the highly concentrated electrosurgical energy 420 (see current field lines) travels from the tip of the cutting element 295 through the tissue 400 to cut the tissue 400 into two distinct halves 430a and 430b. As mentioned above, the number of pulses required to effectively cut the tissue 400 and the intensity of the cutting energy 420 may be determined by measuring the seal thickness and/or tissue impedance and/or based upon an initial calibrating energy pulse which measures similar parameters. A smart sensor 355 (See Fig. 3B) or feedback loop may be employed for this purpose.

As best seen in Fig. 4C, the cutting element 295 may include opposing corner edges 296 having substantially rounded corners. Other cutting element 295 geometries are envisioned as well to create different power concentrations depending upon a particular purpose.

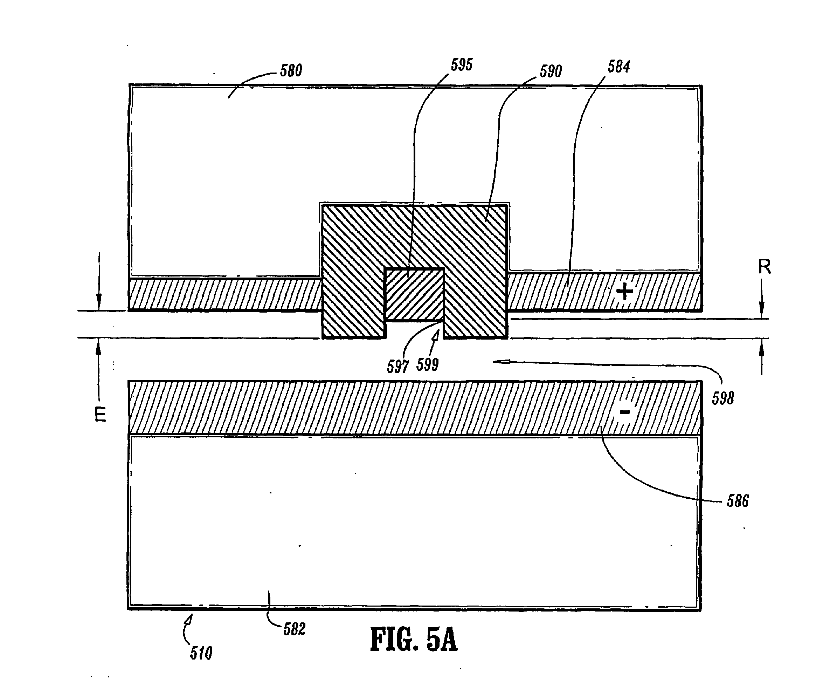

Fig. 5A shows one alternate example of an electrode assembly 500 not in accordance with the claims below, wherein jaw member 582 includes a tissue contacting - surface 584 having an insulator 590 extending along the longitudinal length thereof. In this embodiment, the insulator 590 extends a distance "E" toward the opposing tissue contacting surface 586 to create the stop gap "G" between opposing tissue contacting surfaces 584 and 586 during sealing. A cutting element 595 is recessed within the insulator 590 a distance "R" such that the cutting element 595 does not touch the opposing tissue contacting surface 586 during the sealing and cutting processes. As can be appreciated, tissue contacting surface 586 does not include an insulator oriented opposite the cutting element 595 thus simplifying the overall manufacturing process.

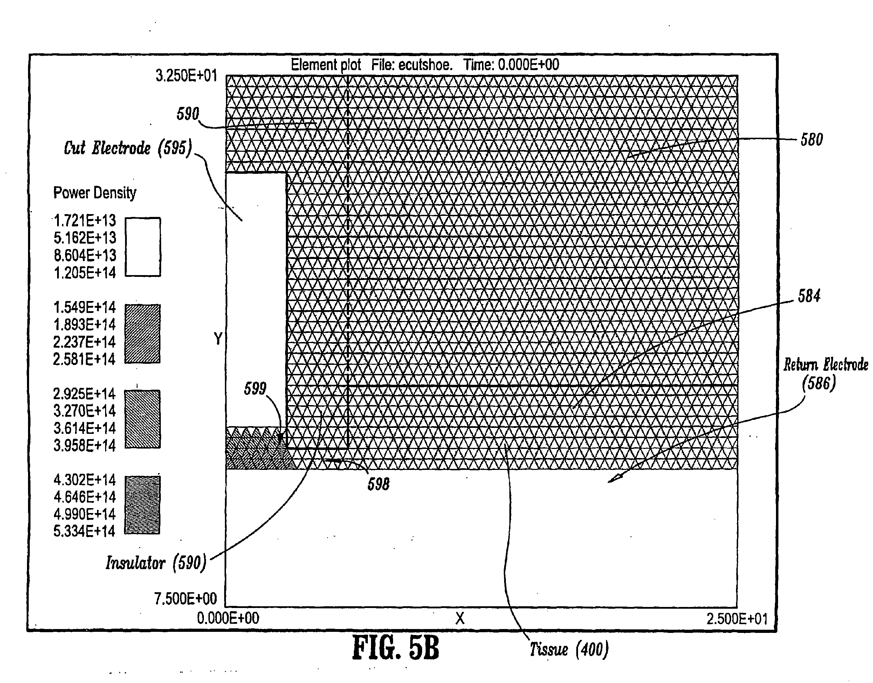

In this electrode assembly, the recessed cutting element 595 is designed to create very high current densities due to the proximity of the insulator 590 relative to the edge 597 of the cutting element 595, i.e., high current densities are created at the insulator/electrode interface 599. As can be appreciated, this particular design of the electrode assembly 510 places the cutting element 595 next to the high pressure insulator/electrode interface 599 (high-pressure clamping zone). As best illustrated in Fig. 5B, a finite element model shows a concentration of high current density near the insulator/electrode interface 599 as a result of placing the cutting element 595 in close proximity to the high-pressure clamping zone 598. It is envisioned that this particular configuration of the cutting element 595 allows for cutting with lower power requirements. Preferably, a pulsing RF output from the ESU 340 may be employed to more effectively cut the tissue 400 as described above.

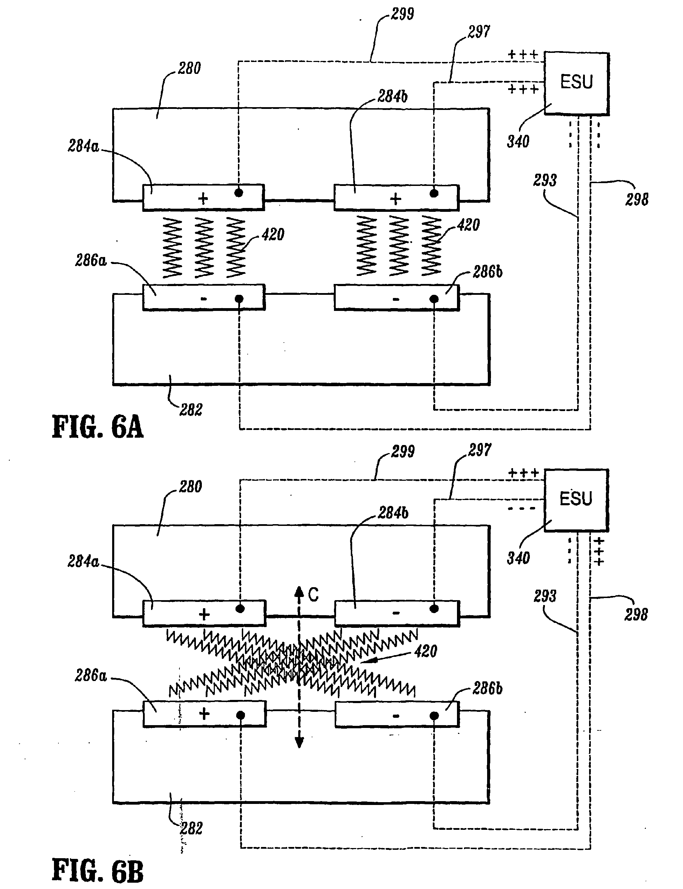

Figs. 6A and 6b show an alternate tissue cutting method wherein the polarities of the sealing electrodes are changed after sealing to effect tissue cutting. More particularly, as best shown in Fig. 6A, a first pair of sealing electrodes 284a and 284b having a first polarity are disposed on jaw member 280 and a second pair of sealing electrodes 286a and 286b having a second polarity are disposed on jaw member 282. A plurality of electrical connections 293, 297, 298 and 299 connect each electrode 286b, 284b, 286a and 284a, respectively, to the electrosurgical generator 340. Electrodes 284a and 286a and electrodes 284b and 286b are positioned in opposing relation relative to one another such that electrosurgical energy 420 can be effectively communicated through tissue 400 when held between the two jaw members 280 and 282.

Upon initial activation and after the tissue 400 is grasped between jaw members 280 and 282, electrosurgical energy is transferred between opposing electrodes 284a and 286a and electrodes 284b and 286b and through the tissue 400 generally in the manner shown in Fig. 6A. As mentioned above, the combination of electrosurgical energy, controlled gap distance between opposing electrodes 284a and 286a and electrodes 284b and 286b and controlled closing pressure will assure a consistent and effective tissue seal.

Once the tissue is effectively sealed between opposing jaw members, the surgeon can opt to cut or divide the tissue along the tissue seal. As can be appreciated, the instrument may be configured to automatically cut the tissue 400 once sealed or the instrument may be configured to permit the surgeon to selectively divide the tissue 400 once sealed. Moreover, it is envisioned that an audible or visual indicator (not shown) may be triggered by a sensor (not shown) to alert the surgeon when an effective seal has been created. The sensor may, for example, determine if a seal is complete by measuring one of tissue impedance, tissue opaqueness and/or tissue temperature. Commonly-owned U.S. Application Serial No. 10/427,832 (US 2004015163) describes several electrical systems which may be employed to provide positive feedback to the surgeon to determine tissue parameters during and after sealing and to determine the overall effectiveness of the tissue seal.

As best seen in Fig. 6B, during the cutting stage the electrical potential of two of the four electrodes, e.g., electrode 284b and electrode 286a, is changed which, as can be appreciated, alters the path of the electrosurgical energy through the tissue 400 when activated. More particularly, an electrical or electromechanical switch (not shown) may be activated after the sealing stage to switch the electrical potential of two of the four electrodes. It is contemplated that the electrical potentials of the electrodes 284a, 284b, 286a and 286b may be reconfigured in any fashion to effect cutting depending upon a particular purpose.

For example and as shown in Fig. 6B, the electrical potentials of electrodes 286a and 284b have been changed to encourage a substantially diagonal electrosurgical cutting path through the tissue 400. More particularly, as the tissue impedance rises, the electrosurgical energy will be directed diagonally towards the electrically opposing electrodes on the opposite jaw member. It is contemplated that the two electrical paths will cross and cut tissue generally along a center line "C".

Preferably, the electrosurgical intensity from each of the electrodes 284a, 284b, 286a and 286b is selectively or automatically controllable to assure consistent and accurate cutting along the centerline "C" in view of the inherent variations in tissue type and/or tissue thickness. Moreover, it is contemplated that the entire surgical process may be automatically controlled such that after the tissue is initially grasped the surgeon may simply activate the forceps to seal and subsequently cut tissue. In this instance, the generator 340 may be configured to communicate with one or more sensors (not shown) to provide positive feedback to the generator 340 during both the sealing and cutting processes to insure accurate and consistent sealing and division of tissue 400. As mentioned above, commonly-owned U.S. Patent Application Serial No. 10/427,832 discloses a variety of feedback mechanisms which may be employed for this purpose.

The present disclosure includes disclosure of a method for sealing and cutting tissue that includes the steps of: providing an electrode assembly, e.g., 210, having a pair of opposing first and second jaw members 280, 282 which are movable relative to one another from a first position wherein the jaw members 280, 282 are disposed in spaced relation relative to one another to a second position wherein the jaw members 280, 282 cooperate to grasp tissue 400 therebetween. Preferably, each of the jaw members 280, 282 includes an electrically conductive tissue contacting surface 284, 286, respectively, which is connected to the ESU 340.

An electrically conductive cutting element 295 is dielectrically disposed within the first tissue contacting surface 284 and the cutting element 295 extends from the first tissue contacting surface 284 towards the second tissue contacting surface 286. Preferably, when the tissue contacting surfaces 284, 286 are closed about the tissue 400, the cutting element 295 creates a gap "G" between the first and second tissue contacting surfaces 284, 286 between about 25 to about 150µm (about 0.001 inches to about 0.006 inches). An insulator is disposed within the second tissue contacting surface 286 in substantially opposing relation to the cutting element.

The method further includes the steps of: actuating the jaw members 280, 282 to grasp tissue 400 between tissue contacting surfaces 284, 286; applying a closure force "F" between tissue contacting surfaces 284, 286 between about 3 kg/cm² to about 16 kg/cm² such that the cutting element 295 creates a gap "G" between the first and second tissue contacting surfaces 284, 286; energizing the first and second tissue contacting surfaces 284, 286 to deliver electrosurgical energy 420 through the tissue 400 to effect a tissue seal 410; and energizing the cutting element 295 and the second tissue contacting surface 286 to deliver electrosurgical energy 420 through the tissue 400 to effectively cut the tissue 400 along the tissue seal 410.

After the step of energizing the first and second tissue contacting surfaces 284, 286 to effect a seal 410, the method may include the step of: utilizing a smart sensor to determine seal quality prior to cutting tissue 400.

From the foregoing and with reference to the various figure drawings, those skilled in the art will appreciate that certain modifications can also be made to the present disclosure without departing from the scope of the present disclosure. For example, it may be preferable to add other features to the forceps 10 or 200, e.g., an articulating assembly to axially displace the electrode assembly 110, 210 relative to the elongated shaft 12, 212. The tissue contacting surfaces 284 and 286 may include radiused edges to facilitate sealing and reduce possible collateral damage to tissue during sealing.

It is also contemplated that the forceps 10 (and/or the ESU 340 used in connection with the forceps 10) may include a second smart sensor or additional feedback mechanism (not shown) which automatically selects the appropriate amount of electrosurgical energy to effectively seal the particularly-sized tissue grasped between the jaw members 280 and 282.

It is envisioned that the outer surface of the jaw members 280 and 282 may include a nickel-based material, coating, stamping and/or metal injection molding which is designed to reduce adhesion between the jaw members 280, 282 (or components thereof) with the surrounding tissue during activation, sealing and cutting. Moreover, it is also contemplated that the tissue contacting surfaces 284 and 286 may be manufactured from one (or a combination of one or more) of the following materials: nickel-chrome, chromium nitride, MedCoat 2000 manufactured by The Electrolizing Corporation of OHIO, inconel 600 and tin-nickel. The tissue contacting surfaces 284, 286 and/or the conductive cutting element 295 may also be coated with one or more of the above materials to achieve the same result, i.e., a "non-stick surface".

Preferably, the non-stick materials are of a class of materials that provide a smooth surface to prevent mechanical tooth adhesions. When utilized on the sealing surfaces 284 and 286 and/or the conductive cutting element 295, these materials provide an optimal surface energy for eliminating sticking due in part to surface texture and susceptibility to surface breakdown due electrical effects and corrosion in the presence of biologic tissues. It is envisioned that these materials exhibit superior non-stick qualities over stainless steel and should be utilized on the forceps 10, 200 in areas where the exposure to pressure and electrosurgical energy can create localized "hot spots" more susceptible to tissue adhesion. As can be appreciated, reducing the amount that the tissue "sticks" during sealing improves the overall efficacy of the instrument.

As mentioned above, the non-stick materials may be manufactured from one (or a combination of one or more) of the following "non-stick" materials: nickel-chrome, chromium nitride, MedCoat 2000, Inconel 600 and tin-nickel. For example, high nickel chrome alloys, Ni200, Ni201 (~100% Ni) may be made into the sealing surfaces 284, 286 or the cutting element 295 by metal injection molding, stamping, machining or any like process. Also and as mentioned above, the tissue sealing surfaces 284 and 286 and/or the conductive cutting element 295 may also be "coated" with one or more of the above materials to achieve the same result, i.e., a "non-stick surface". For example, Nitride coatings (or one or more of the other above-identified materials) may be deposited as a coating on another base material (metal or nonmetal) using a vapor deposition manufacturing technique.

One particular class of materials disclosed herein has demonstrated superior non-stick properties and, in some instances, superior seal quality. For example, nitride coatings which include, but not are not limited to: TiN, ZrN, TiAlN, and CrN are preferred materials used for non-stick purposes. CrN has been found to be particularly useful for non-stick purposes due to its overall surface properties and optimal performance. Other classes of materials have also been found to reducing overall sticking. For example, high nickel/chrome alloys with a Ni/Cr ratio of approximately 5:1 have been found to significantly reduce sticking in bipolar instrumentation. One particularly useful non-stick material in this class is Inconel 600. Bipolar instrumentation having sealing surfaces 284 and 286 made from or coated with Ni200, Ni201 (~100% Ni) also showed improved non-stick performance over typical bipolar stainless steel electrodes.

By way of example, chromium nitride may be applied using a physical vapor deposition (PVD) process that applies a thin uniform coating to the entire conductive surface. This coating produces several effects: 1) the coating fills in the microstructures on the metal surface that contribute to mechanical adhesion of tissue to surfaces; 2) the coating is very hard and is a non-reactive material which minimizes oxidation and corrosion; and 3) the coating tends to be more resistive than the base material causing conductive surface heating which further enhances desiccation and seal quality.

The Inconel 600 coating is a so-called "super alloy" which is manufactured by Special Metals, Inc. located in Conroe Texas. The alloy is primarily used in environments which require resistance to corrosion and heat. The high Nickel content of Inconel makes the material especially resistant to organic corrosion. As can be appreciated, these properties are desirable for bipolar electrosurgical instruments which are naturally exposed to high temperatures, high RF energy and organic matter. Moreover, the resistivity of Inconel is typically higher than the base conductive material which further enhances desiccation and seal quality.

As disclosed herein the present invention facilitates the transfer of electrosurgical energy though opposing electrically conductive sealing surfaces having different electrical potentials to effect vessel sealing. However, it is also contemplated that the presently disclosed embodiments discussed herein may be designed to seal the tissue structure using so-called "resistive heating" whereby the tissue contacting surfaces 284 and 286 are not necessarily electrically conductive surfaces. Rather, each of the surfaces 284 and 286 is heated much like a conventional "hot plate" such that the surfaces 284 and 286 cooperate to seal the tissue upon contact (or upon activation of a switch (not shown) which selectively heats each surface 284 and 286 upon activation).

Preferably, the presently disclosed forceps 10, 200 is designed to electrically- couple to a foot switch (not shown) which allows the surgeon to selectively control the electrosurgical energy transferred to the tissue either to effect sealing and/or cutting. A handswitch (not shown) may also be utilized. As can be appreciated, locating a handswitch on the forceps 10, 200 has many advantages. For example, the handswitch reduces the amount of electrical cable in the operating room and virtually eliminates the possibility of activating the wrong instrument or feature during a surgical procedure due to "line-of-sight" activation.

It is also contemplated that cutting element 295 may be dimensioned as a cutting wire which is selectively activatable by the surgeon to divide the tissue 400 after sealing. More particularly, a wire is mounted within the insulator 290 between the jaw members 280 and 282 and is selectively energizable upon activation of the switch 350.

It is envisioned that the electrode assembly 110, 210 could be selectively detachable (i.e., reposable) from the shaft 12, 212, respectively, depending upon a particular purpose. Alternatively, the entire instrument could be disposable. For example, it is contemplated that specific forceps 10, 200 could be configured for different tissue types or thicknesses. Moreover, it is envisioned that a reusable forceps 10, 200 could be sold as a kit having different electrodes assemblies 110, 210 for different tissue types. The surgeon simply selects the appropriate electrode assembly for a particular tissue type.

It is also envisioned that a variable resistor could be employed to regulate the electrosurgical energy to effect sealing and or cutting a particular tissue type. The variable resistor could be coupled to a sensor which determines tissue type, tissue impedance, tissue moisture, tissue thickness, etc. and regulates the appropriate electrosurgical energy intensity by automatically adjusting the variable resistor to seal and /or the cut tissue.

Various embodiments of stop member configurations and stop gap actuators are disclosed in commonly-owned PCT Patent Application Serial Nos. PCT US 02/01890 (WO 02 080799) and PCT/US01/11413 (WO 02 080796).

## Claims

1. An electrode assembly (110) for use with an instrument (10) for sealing vessels, the electrode assembly comprising:
a pair of opposing first (280) and second (282) jaw members being movable relative to one another from a first position wherein the jaw members are disposed in spaced relation relative to one another to a second position wherein the jaw members cooperate to grasp tissue therebetween;
each jaw member including an electrically conductive tissue contacting surface (284 , 286), each tissue contacting surface being connected to a source (340) of electrosurgical energy such that the tissue contacting surfaces are capable of conducting electrosurgical energy through the tissue held therebetween to effect a seal;
the first jaw member including an electrically conductive cutting element (295) within the first tissue contacting surface and the second jaw member including an insulator (292) disposed therein, the insulator being disposed in substantially opposing relation to the cutting element; wherein the cutting element and the first tissue contacting surface are independently activatable by the surgeon,
**characterised in that**:
the cutting element is so embedded in an insulator (290) that it extends from the first tissue contacting surface towards the second tissue contacting surface to create a gap (B) between the tissue contacting surfaces during sealing when the jaw members are in the second position.

2. An electrode assembly according to claim 1, wherein the cutting element is substantially dull and only capable of cutting tissue through electrosurgical activation.

3. An electrode assembly according to any preceding claim further comprising a smart sensor (355) for determining seal quality prior to cutting.

4. An electrode assembly according to claim 3, wherein the smart sensor includes an audible indicator (360) for indicating seal quality.

5. An electrode assembly according to any preceding claim, wherein the smart sensor includes a visual indicator (360) for indicating seal quality.

6. An electrode assembly according to claim 3, 4 or 5, wherein the smart sensor determines seal quality based upon a drop in electrical current through the tissue.

7. An electrode assembly according to claim 3, 4 or 5, wherein the smart sensor determines seal quality based upon tissue impedance.

8. An electrode assembly according to claim 3, 4 or 5, wherein the smart sensor determines seal quality based upon tissue temperature.

9. An electrode assembly according to any preceding claim further comprising a smart sensor (355) for determining various tissue parameters to control the electrosurgical energy supplied to the cutting element during cutting.

10. An electrode assembly according to claim 9 so arranged that, in use, after the tissue seal is created, the smart sensor measures at least one of tissue thickness, tissue moisture, tissue density and tissue impedance to control the electrosurgical energy supplied to the cutting element during cutting.

11. An electrode assembly according to claim 9 or 10 so arranged that, is use, after the tissue seal is created, the electrosurgical energy source sends a calibrating pulse through the tissue to enable the smart sensor to measure various tissue parameter to control the electrosurgical energy supplied to the cutting element during cutting.

12. An electrode assembly according to any preceding claim wherein, is use, the electrosurgical energy source delivers electrosurgical energy in a pulse-like matter to effect at least one of cutting and sealing.

13. An electrode assembly according to any preceding claim further comprising a smart sensor (355) which includes a variable resistor to regulate the electrosurgical energy during both the sealing and cutting processes.

14. An electrode assembly according to any preceding claim further comprising a first switch (350a) for energizing the opposing first and second tissue contacting surfaces to effect tissue sealing and a second switch (350b) for energizing the cutting element and the second tissue contacting surface to effect tissue cutting.

## Patentansprüche

1. Elektrodenanordnung (110) zur Verwendung mit einem Instrument (10) zum Abdichten von Gefäßen, wobei die Elektrodenanordndung umfasst:
ein Paar von gegenüberliegenden ersten (280) und zweiten (282) Klauenelementen, die relativ zueinander von einer ersten Position, bei der die Klauenelemente in beabstandeter Beziehung relativ zueinander angeordnet sind, zu einer zweiten Position, bei der die Klauenelemente zusammenwirken, um Gewebe dazwischen zu greifen, beweglich sind;
wobei jedes Klauenelement eine elektrisch leitende Gewebekontaktfläche (284, 286) umfasst, und jede Gewebekontaktfläche mit einer Quelle (340) elektrochirurgischer Energie verbunden ist, so dass die Gewebekontaktflächen in der Lage sind, elektrochirurgische Energie durch das zwischen ihnen gehaltene Gewebe zu leiten und so eine Abdichtung zu bewirken;
wobei das erste Klauenelement ein elektrisch leitendes Schneidelement (295) in der ersten Gewebekontaktfläche umfasst und das zweite Klauenelement einen darin angeordneten Isolator (292) umfasst, wobei der Isolator in im Wesentlichen gegenüberliegenden Verhältnis zum Schneidelement angeordnet ist; wobei das Schneidelement und die erste Gewebekontaktfläche durch den Chirurgen unabhängig betätigbar sind,
**dadurch gekennzeichnet, dass**:
das Schneidelement so in einem Isolator (290) eingebettet ist, dass es sich von der ersten Gewebekontaktfläche zur zweiten Gewebekontaktfläche erstreckt, um einen Spalt (B) zwischen den Gewebekontaktflächen während des Abdichtens zu erzeugen, wenn die Klauenelemente sich in der zweiten Position befinden.

2. Elektrodenanordnung nach Anspruch 1, wobei das Schneidelement im Wesentlichen stumpf ist und lediglich in der Lage ist, Gewebe durch elektrochirurgische Betätigung zu schneiden.

3. Elektrodenanordnung nach einem der vorangehenden Ansprüche, weiter einen intelligenten Sensor (355) zur Bestimmung der Qualität der Abdichtung vor dem Schneiden umfassend.

4. Elektrodenanordnung nach Anspruch 3, wobei der intelligente Sensor einen hörbaren Indikator (360) zum Anzeigen der Qualität der Abdichtung umfasst.

5. Elektrodenanordnung nach einem der vorangehenden Ansprüche, wobei der intelligente Sensor einen visuellen Indikator (360) zum Anzeigen der Qualität der Abdichtung umfasst.

6. Elektrodenanordnung nach Anspruch 3, 4 oder 5, wobei der intelligente Sensor die Qualität der Abdichtung basierend auf einem Abfall des elektrischen Stroms durch das Gewebe bestimmt.

7. Elektrodenanordnung nach Anspruch 3, 4 oder 5, wobei der intelligente Sensor die Qualität der Abdichtung basierend auf der Gewebeimpendanz bestimmt.

8. Elektrodenanordnung nach Anspruch 3, 4 oder 5, wobei der intelligente Sensor die Qualität der Abdichtung basierend auf der Gewebetemperatur bestimmt.

9. Elektrodenanordnung nach einem der vorangehenden Ansprüche, weiter einen intelligenten Sensor (355) zur Bestimmung von verschiedenen Gewebeparametern umfassend, um die dem Schneidelement während des Schneidens zugeführte elektrochirurgische Energie zu steuern.

10. Elektrodenanordnung nach Anspruch 9, die so angeordnet ist, dass im Einsatz, nachdem die Gewebeabdichtung erzeugt wurde, der intelligente Sensor die Gewebedicke und/oder die Gewebefeuchtigkeit und/oder die Gewebedichte und/oder die Gewebeimpendanz misst, um die dem Schneidelement während des Schneidens zugeführte elektrochirurgische Energie zu steuern.

11. Elektrodenanordnung nach Anspruch 9 oder 10, die so angeordnet ist, dass im Einsatz, nachdem die Gewebeabdichtung erzeugt wurde, die elektrochirurgische Energiequelle einen Kalibrierimpuls durch das Gewebe aussendet, um den intelligenten Sensor in die Lage zu versetzen, verschiedene Gewebeparameter zu messen und so die dem Schneidelement während des Schneidens zugeführte elektrochirurgische Energie zu steuern.

12. Elektrodenanordnung nach einem der vorangehenden Ansprüche, wobei im Einsatz die elektrochirurgische Energiequelle elektrochirurgische Energie auf impulsartige Weise zuführt, um ein Schneiden und/oder Abdichten zu bewirken.

13. Elektrodenanordnung nach einem der vorangehenden Ansprüche, weiter einen intelligenten Sensor (355) umfassend, der einen variablen Widerstand aufweist, um die elektrochirurgische Energie sowohl während des Abdichtvorgangs als auch des Schneidvorgangs zu regeln.

14. Elektrodenanordnung nach einem der vorangehenden Ansprüche, weiter einen ersten Schalter (350a) zur Versorgung der gegenüberliegenden ersten und zweiten Gewebekontaktfläche mit Energie umfassend, um eine Gewebeabdichtung zu bewirken, und einen zweiten Schalter (350b) zur Versorgung des Schneidelements und der zweiten Gewebekontaktfläche umfassend, um einen Schnitt des Gewebes zu bewirken.

## Revendications

1. Agencement d'électrode (110) pour utilisation avec un instrument (10) pour souder des vaisseaux, l'agencement d'électrode comprenant :
une paire de premier (280) et second (282) éléments de mâchoire déplaçables l'un relativement à l'autre d'une première position où les éléments de mâchoire se trouvent dans une relation espacée l'un relativement à l'autre à une seconde position dans laquelle les éléments de mâchoire coopèrent pour saisir le tissu entre eux ;
chaque élément de mâchoire comprenant une surface électriquement conductrice (284, 286) venant en contact avec le tissu, chaque surface venant en contact avec le tissu étant reliée à une source (340) d'énergie électro-chirurgicale de telle sorte que les surfaces venant en contact avec le tissu sont aptes à conduire l'énergie électro-chirurgicale à travers le tissu retenu entre elles pour effectuer une soudure ;
le premier élément de mâchoire comprenant un élément de coupe électriquement conducteur (295) dans la première surface venant en contact avec le tissu, et le second élément de mâchoire comprenant un isolateur (292) disposé dans celui-ci, l'isolateur étant disposé en une relation sensiblement opposée à l'élément de coupe ; où l'élément de coupe et la première surface venant en contact avec le tissu peuvent être activés indépendamment par le chirurgien,
**caractérisé en ce que**
l'élément de coupe est noyé dans un isolateur (290) de telle sorte qu'il s'étend de la première surface venant en contact avec le tissu vers la seconde surface venant en contact avec le tissu pour créer un espace (B) entre les surfaces venant en contact avec le tissu pendant la soudure lorsque les éléments de mâchoire se trouvent dans la seconde position.

2. Agencement d'électrode selon la revendication 1, où l'élément de coupe est sensiblement émoussé et est seulement apte à couper le tissu par une activation électro-chirurgicale.

3. Agencement d'électrode selon l'une des revendications précédentes, comprenant en outre un capteur intelligent (355) pour déterminer la qualité de la soudure avant la coupe.

4. Agencement d'électrode selon la revendication 3, où le capteur intelligent comprend un indicateur audible (360) pour indiquer la qualité de la soudure.

5. Agencement d'électrode selon l'une des revendications précédentes, où le capteur intelligent comprend un indicateur visuel (360) pour indiquer la qualité de la soudure.

6. Agencement d'électrode selon la revendication 3, 4 ou 5, où le capteur intelligent détermine la qualité de la soudure sur la base d'une chute du courant électrique à travers le tissu.

7. Agencement d'électrode selon la revendication 3, 4 ou 5, où le capteur intelligent détermine la qualité de la soudure sur la base de l'impédance du tissu.

8. Agencement d'électrode selon la revendication 3, 4 ou 5, où le capteur intelligent détermine la qualité de la soudure sur la base de la température du tissu.

9. Agencement d'électrode selon l'une des revendications précédentes, comprenant en outre un capteur intelligent (355) pour déterminer divers paramètres de tissu afin de régler l'énergie électro-chirurgicale fournie à l'élément de coupe pendant la coupe.

10. Agencement d'électrode selon la revendication 9, agencé de telle sorte que, en utilisation, après que la soudure de tissu a été créée, le capteur intelligent mesure au moins l'une parmi l'épaisseur du tissu, l'humidité du tissu, la densité du tissu et l'impédance du tissu pour régler l'énergie électro-chirurgicale appliquée à l'élément de coupe pendant la coupe.

11. Agencement d'électrode selon la revendication 9 ou 10 agencé de telle sorte que, en cours d'utilisation, après que la soudure de tissu a été créée, la source d'énergie électro-chirurgicale transmet une impulsion de calibrage à travers le tissu pour permettre au capteur intelligent de mesurer divers paramètres du tissu afin de régler l'énergie électro-chirurgicale fournie à l'élément de coupe pendant la coupe.

12. Agencement d'électrode selon l'une des revendications précédentes, dans lequel, en cours d'utilisation, la source d'énergie électro-chirurgicale transmet de l'énergie électro-chirurgicale sous forme d'impulsions pour effectuer au moins l'une de la coupe et de la soudure.

13. Agencement d'électrode selon l'une des revendications précédentes, comprenant en outre un capteur intelligent (355) qui comprend une résistance variable pour régler l'énergie électro-chirurgicale à la fois pendant les processus de soudure et de coupe.

14. Agencement d'électrode selon l'une des revendications précédentes, comprenant en outre un premier commutateur (350a) pour exciter les première et seconde surfaces opposées venant en contact avec le tissu pour effectuer la soudure du tissu, et un second commutateur (350b) pour exciter l'élément de coupe et la deuxième surface venant en contact avec le tissu pour effectuer la coupe du tissu.
